# EUROPEAN PATENT SPECIFICATION

(11) **EP 4 440 913 B1**
(45) Date of publication and mention of the grant of the patent: **23.04.2025**
(21) Application number: 22813758.4
(22) Date of filing: 24.11.2022
(51) Int. Cl.: B62J 45/421, B62J 45/41, B62M 1/36, B62M 3/08, A61B 5/103, A63B 24/00, G01L 5/00

(54) **SYSTEM AND METHOD OF MEASUREMENT FOR BICYCLES**
SYSTEM UND VERFAHREN ZUR MESSUNG FÜR FAHRRÄDER
SYSTÈME ET PROCÉDÉ DE MESURE POUR BICYCLETTES

(30) Priority: 30.11.2021 IT 202100030326
(43) Date of publication of application: 09.10.2024
(73) Proprietor: FAVERO ELECTRONICS S.R.L., 31030 Arcade (TV) (IT)
(72) Inventor: FAVERO, Stefano, 31030 ARCADE (TV) (IT)
(74) Representative: Studio Torta S.p.A.
(86) International application number: PCT/IB2022/061400
(87) International publication number: WO 2023/100044

(56) References cited:
- EP-A1- 2 783 729
- US-A- 5 027 303
- US-A1- 2013 205 916
- US-A1- 2016 023 081
- US-A1- 2018 321 096

## Description

### Cross-Reference to Related Applications

This Patent Application claims priority from Italian Patent Application No. 102021000030326 filed on November 30, 2021.

### Technical Field

The present invention relates to a system and method of measurement for bicycles. In particular, the present invention concerns the determination of performance data based on the measurement of the force exerted by a cyclist during one or more pedal strokes.

### Prior Art

Electronic measuring systems and apparatuses for bicycles, generally referred to as "power meter apparatuses", which are mounted on a bicycle pedal or pedal-crank in order to determine a series of performance data that characterize the cyclist's physical performance during his/her pedalling, are well known.

The power meter apparatuses mentioned above are commonly equipped with sensors that measure a number of physical quantities, such as for example the force exerted by the cyclist on the pedal, the pedal rotation speed and cadence, and with an electronic unit that processes these measured physical quantities in order to determine the aforementioned performance data, usually also referred to with the technical term "metrics".

The metrics currently determined by the power meter apparatuses described above are manifold. The performance data of the most widespread metrics include, for example: total power, power phase, torque effectiveness, power smoothness, and other physical quantities that are directly or indirectly related to the force exerted by the user, i.e., the cyclist.

Although the current metrics provide a wide range of performance data that can be used to enable technical and athletic assessments of the cyclist's performance to be made, there is currently a need, especially in sectors associated with competitive cycling, to be able to obtain more specific, useful, and selective performance data, through which technical analyses can be made to gain more information on the cyclist's pedal stroke.

The solutions described in US2015/306458A1, US2018/321096A1,US2013/205916A1,US5027303A, US2016/023081A1 and EP2783729A1 are also known. Document US 2013/205916 A1 discloses the preamble of claim 1.

### Description of the Invention

The object of the present invention is therefore to provide a system and a method capable of meeting the above need.

This object is achieved by the present invention in that it relates to an electronic measuring system for bicycles provided as defined in the related appended system claims.

According to the present invention, there is further provided a method of measurement for bicycles as defined in the related appended method claims.

The claims describe preferred embodiments of the present invention forming an integral part of the present specification.

### Brief Description of the Drawings

The present invention will now be described with reference to the accompanying drawings, which illustrate a non-limiting embodiment thereof, wherein:
- Figure 1 is a perspective view, with parts on an enlarged scale, of an electronic measuring system for bicycles provided according to the teachings of the present invention,
- Figure 2 shows a block diagram of the electronic measuring system for bicycles provided according to the teachings of the present invention,
- Figures 3 and 4 show respective pedalling circumferences and pedalling arcs,
- Figure 5 shows a user interface device of the electronic measuring system provided according to the teachings of the present invention,
- Figures 6 and 7 show a pedal movement within a pedalling arc,
- Figure 8 shows a user interface device of the electronic measuring system provided according to a possible embodiment of the present invention,
- Figure 9 shows a flowchart of the operations implemented by the electronic measuring system according to the present invention.

### Preferred Embodiments of the Invention

The present invention will now be described in detail with reference to the accompanying drawings in order to allow a skilled person to implement it and use it. Various modifications to the described embodiments will be readily apparent to those skilled in the art and the general principles described may be applied to other embodiments and applications without however departing from the protective scope of the present invention as defined in the appended claims. Therefore, the present invention should not be construed as limited to the embodiments described and illustrated herein, but it must be given the broadest protective scope consistent with the principles and features described and claimed herein.

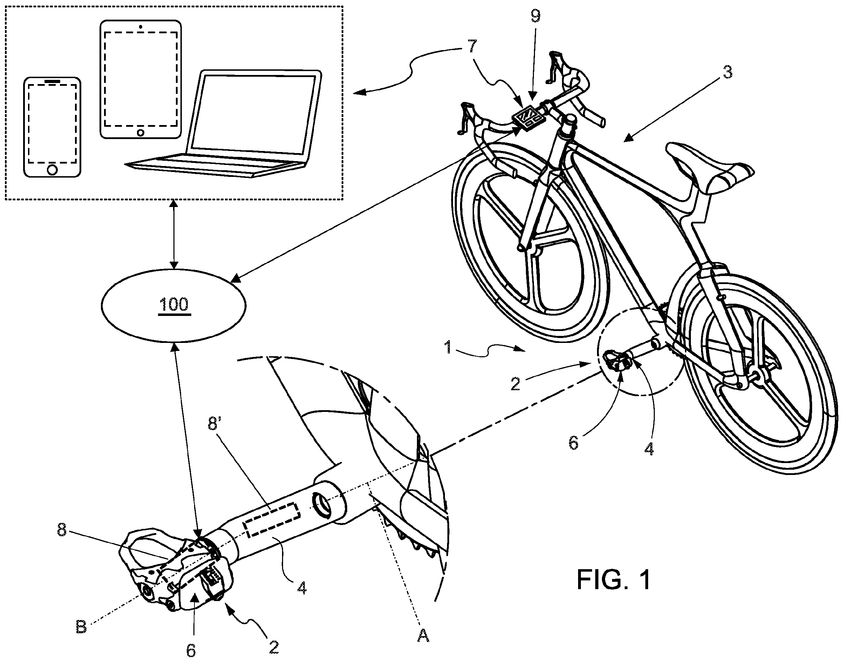

With reference to Figure 1, number 1 indicates, as a whole, an electronic measuring system for bicycles. As will be described in detail below, the electronic measuring system 1 is configured to provide metrics comprising performance data of a user during one or more pedal strokes.

The metrics, i.e., the performance data, are determined by the electronic measuring system 1 on the basis of at least one force exerted by a user (cyclist) during at least one pedal stroke, in a condition where pedalling causes the bicycle 3 to move on a support surface, for example, a road or the like.

It is understood that the present invention is not limited to metrics/performance data determined by the electronic measuring system 1 on the basis of the pedal stroke performed by the user through a single pedal 2, but can be applied, in much the same way as described below, to determine the user's metrics/performance data on the basis of the pedal strokes performed by the user on the two pedals 2 (right-hand pedal and left-hand pedal) of the bicycle 3.

The bicycle 3 is of a known type and, as it is not the object of the present invention, will not be further described except to specify (Figure 1) that it comprises a frame, a handlebar, two pedal-cranks 4 coupled to a drive assembly mounted on the hub of the frame of the bicycle 3 so that they rotate around a rotation axis A, and two pedals 2 which are coupled to their respective pedal-cranks 4. The pedals 2 of the bicycle 3 each comprise a pedal pin (not shown) which is coupled to the pedal-crank 4 and extends along an axis B orthogonal to the axis of the pedal-crank 4. The pedal 2 also comprises a pedal body 6, which is coupled to the pedal pin so that it rotates freely around the axis B and is capable of forming, in use, the support/rest structure for the user's foot and/or the structure for the fastening of his/her footwear.

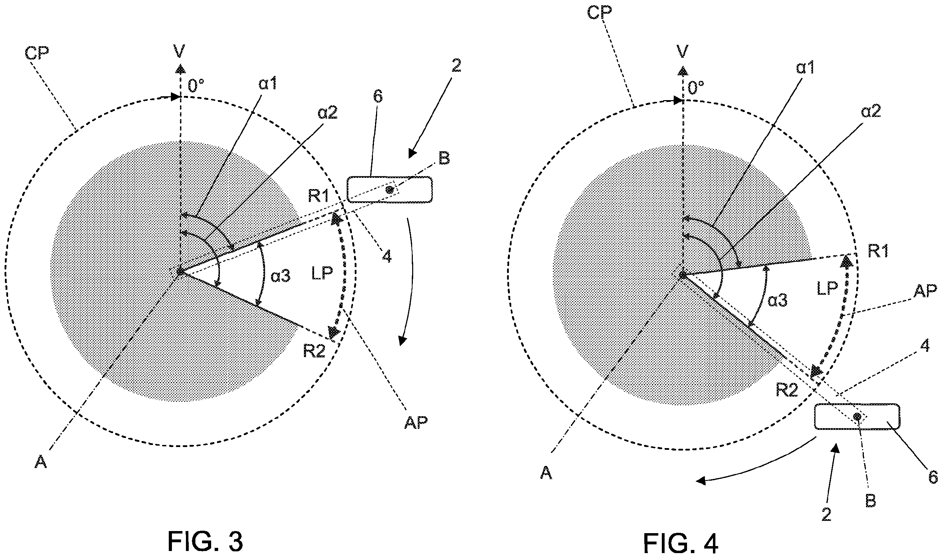

With reference to Figures 3 and 4, in use, each pedal 2 describes/follows a substantially circular pedalling trajectory, which defines a pedalling circumference CP when the respective pedal-crank 4 makes one full revolution around the rotation axis A, covering a rotation angle of 360° measured in relation to a reference axis V, represented by a vertical axis corresponding to a rotation angle of the pedal-crank 4 of 0°.

With reference to Figures 3 and 4, in the following discussion, the term "pedalling arc" refers to a section of the pedalling circumference CP which, in turn, is defined by the pedalling trajectory covered by the pedal 2 during a full revolution (360°). In other words, the pedalling arc indicated with AP in the attached figures, is a segment or portion of the circumference that joins two points of the pedalling circumference CP, precisely defining the ends of the pedalling arc AP itself. In Figures 3 and 4, the ends of the pedalling arc AP of the pedalling circumference CP are joined to the centre of the circumference by two radii R1 and R2. In the examples shown, the first radius R1 forms an angle α1 with the reference axis V, and the second radius R2 forms an angle α2>α1 with the same reference axis V. The angular difference α2-α1 defines a pedalling angle α3 which is comprised between the two radii R1, R2 and defines the angular width of the pedalling arc AP. The pedalling arc AP is characterized by a pedalling length LP less than the length of the pedalling circumference CP. According to the present invention, the pedalling arc AP also has a pedalling angle α3 less than 360°.

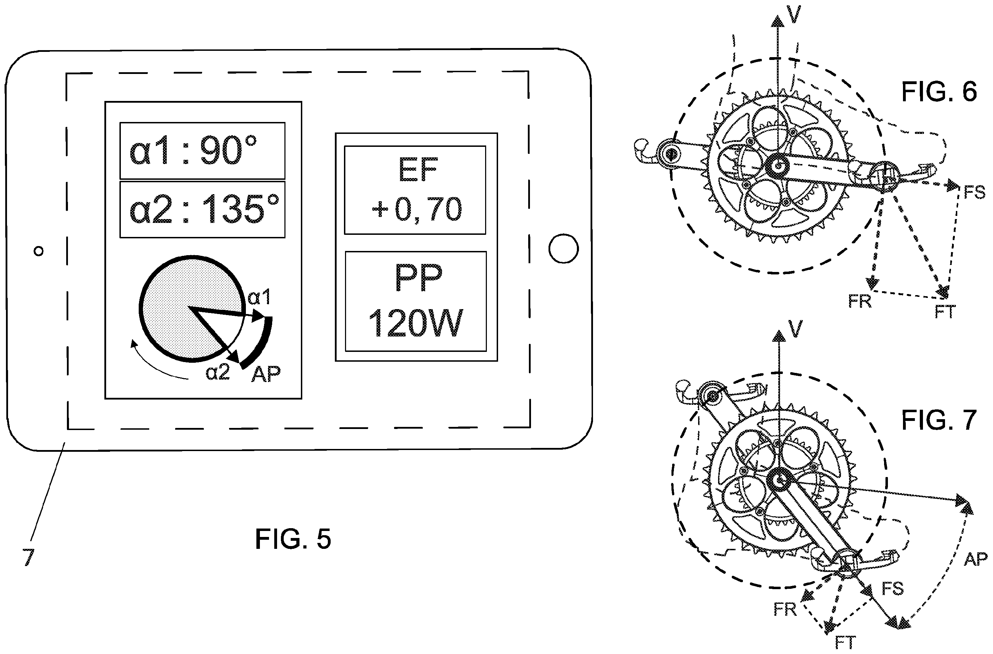

In the following discussion, the term "resultant force" refers to the total force applied by the user to the pedal 2. In Figures 6 and 7, the resultant force is indicated by FT.

In the following discussion, the term "effective force" refers to the component of the resultant force FT acting radially to the pedal-crank 4 at the point of application of the pedal 2 (tangentially to the pedalling circumference CP). It is understood that the effective force, shown in Figures 6 and 7 by FR, since it is a vector, can be a positive effective force (when its direction agrees with the direction of rotation of the pedal-crank 4 around the axis A - clockwise from the side of the bicycle equipped with the pedal-crank), or a negative effective force when its direction is opposite to the direction of rotation of the pedal-crank around the axis A.

In the following discussion, the term "axial force" refers to the component of the total force FT oriented along the axis of the pedal-crank 4. In Figures 6 and 7, the axial force is indicated by FS.

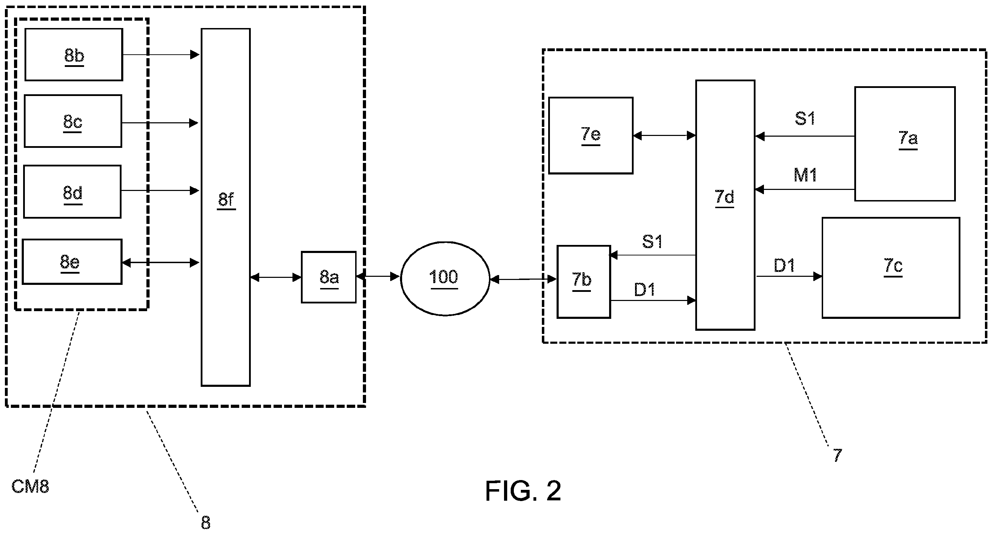

With reference to a preferred embodiment shown in Figures 1 and 2, the electronic system 1 comprises an electronic user interface device 7, which is configured to allow a user (i.e., a cyclist) to set and/or define/select at least one pedalling arc AP in the pedalling circumference CP of a pedal 2. The user interface device 7 is configured to provide a user-command S1 containing command-data indicative of the pedalling arc AP of the pedal 2 set and/or defined/selected by the user.

The electronic system 1 further comprises at least one electronic power meter apparatus 8, which is mounted on the pedal 2. It is also understood that, according to a possible embodiment, alternatively and/or additionally, the power meter apparatus (shown in Figure 1 with 8' for clarity) may be arranged on the pedal-crank 4.

It is also understood that the electronic system 1 may comprise two power meter apparatuses 8 mounted on the respective pedals 2 and/or on the respective pedal-cranks 4; in this case, the user interface device 7 is configured to allow a user to define/select at least one pedalling arc AP in the pedalling circumference CP for each of the two pedals 2.

The power meter apparatus 8 is configured to: be operationally connected to the user interface device 7 to receive the user-command S1 containing the pedalling-arc AP set and/or selected by the user, measure at least one force exerted by the user during pedalling, when the pedal 2 is moving in the set pedalling arc AP, determine at least one measurement quantity associated with the force measured in the set pedalling arc AP, and determine the user's performance data in the set pedalling arc AP on the basis of the determined measurement quantity.

According to the preferred embodiment shown in Figure 1, the power meter apparatus 8 is configured to measure at least one force exerted by a user on the pedal 2 in the pedalling circumference CP or in the set pedalling arc AP, determine one or more measurement quantities based on the force measured on the pedal 2 when the pedal 2 moves within the pedalling arc AP set and/or selected/established by the user, and provide the pedalling performance data associated with the force measured in the set pedalling arc AP, on the basis of said one or more determined measurement quantities.

The technical effect obtained is to enable a user to greatly deepen his/her knowledge of his/her performance in a section, i.e., a specific sector, contained within the pedalling circumference CP. In this way, the parameters characterizing the pedalling movement performed by the user can be specifically monitored and/or mapped within the pedalling arc set by the user, so as to enable targeted technical processing that allows said movement to be better analysed in order to correct it in real time if the observed performance deviates from the required performance.

According to a preferred embodiment shown in Figure 1, the user interface device 7 can conveniently be included in, or be, a cycle computer placed on the bicycle 3 (on the handlebar in Figure 1).

However, it is understood that the present invention is not limited to the user interface device 7 of a cycle computer 9, but may include, as shown in Figure 1, additionally and/or alternatively, a user interface device 7 included in, or corresponding to, any computer device such as for example a smart-phone, tablet, or laptop, or any other similar electronic computing and/or communication device.

According to a preferred embodiment, the user interface device 7 can be configured to allow a user to set, i.e., adjust/vary, the pedalling arc AP and is designed to code the adjustment/variation made in the command data contained in the command signal S1 supplied to the power meter apparatus 1. According to this preferred embodiment, the pedalling arc AP is not predefined and is therefore adjusted as desired by the user.

The adjustment that can be carried out by the user through the user interface device 7 can be selective and include: the variation of the length LP of the pedalling arc AP, and/or the variation of the angular width α3 of the pedalling arc AP, the variation of the position of the end points of the pedalling arc AP in the circumference, for example, by varying the angle α1 and/or the angle α2. This adjustment can be coded in the command data contained in the command signal S1. The command data may thus contain the geometrical parameters described above that characterize the pedalling arc AP defined by the adjustment made.

The pedalling arc AP may conveniently have an angular width α3 different from 45°. The pedalling arc AP may conveniently have an angular width α3 less than 45°. The angle α1 or the angle α2 of the pedalling arc AP may each have a value that is different from the following values: 0°, 45°, 90°, 135°, 180°, 225°, 270°, 315°, 360°.

The user interface device 7 is configured so that it does not communicate to the user performance data relating to pedalling arcs AP of the pedalling circumference CP which are not set by the user.

Alternatively, and/or additionally, the user interface device 7 can also be configured to allow a user to selectively set, i.e., select a pedalling arc AP from a plurality of predetermined pedalling arcs AP, and code the selected arc in the command data of the user-command signal S1. Each of the predetermined and selectable pedalling arcs AP may have different geometrical parameters from those characterizing the rest of the selectable pedalling arcs AP.

With reference to Figure 2, according to a possible embodiment, the user interface device 7 may comprise: an operating unit 7a by means of which the user gives the above command data via the command signal S1. The operating unit 7a may include, for example, a panel and/or a keypad, and/or a display. The display can conveniently be a touch-screen display or the like. The operating unit 7a may be configured to allow the user to set and/or define/select a pedalling arc AP using manual and/or voice commands, or any other similar type of command.

The user interface device 7 further comprises a communication unit 7b which is configured to perform bidirectional communication, preferably wireless through a communication system 100, with the power meter apparatus 8 to provide it with the command signal S1 and/or to receive pedalling performance data therefrom. The communication system 100 can be a short-range wireless system (WPAN) (Bluetooth communication, or the like) and/or a long-range wireless system (cellular telephone communication, or the like).

With reference to Figure 2, according to a possible embodiment, the user interface device 7 may further comprise an output unit 7c which is operationally connected to the communication unit 7b, in order to receive pedalling performance data therefrom, and is configured to communicate the pedalling performance data to the user.

With reference to Figure 2, according to a possible embodiment, the user interface device 7 may further comprise a processing unit 7d which controls the operation of all the above-described units in the user interface device 7.

According to a convenient embodiment shown in Figure 2, the electronic power meter apparatus 8 may comprise a communication module 8a designed to communicate with the communication unit 7b of the user interface device 7 through the communication system 100 in order to receive the command signal S1 and provide the pedalling performance data.

According to a convenient embodiment shown in Figure 2, the electronic power meter apparatus 8 may further comprise a measurement circuit CM8 equipped with a sensor device 8b configured to measure a force exerted by the user on the pedal 2.

With reference to the preferred embodiment shown in Figures 1, 2, 6 and 7, the force measured by the power meter apparatus 8 mounted on the pedal 2 may optionally include one or more of the following forces: the resultant force FT and/or the effective force FR and/or the axial force FS.

It is understood that in the case where the power meter apparatus 8 is mounted on the pedal-crank 4, the measured force may include the force orthogonal to the axis of the pedal-crank 4 and/or the force coaxial with the axis of the pedal-crank 4.

As shown in Figure 2, the sensor device 8b is designed to provide the measured force(s). The sensor device 8b may preferably comprise strain gauges stably coupled to the pedal pin of the pedal 2 (or to the pedal-crank 4) in order to measure the deformation thereof. The strain gauges can be connected to each other so as to form one or more Wheatstone bridge circuits.

The sensor device 8b can be made, for example, as described in the Applicant's Italian patent applications no. 102019000022536, 102017000052407, 102020000017662.

The measurement circuit CM8 may further comprise one or more sensor devices 8c designed to measure the angular speed of rotation ω of the pedal-crank 2 instant by instant. The sensor devices 8c may include, for example, accelerometers and/or gyroscopes with one or more axes.

The measurement circuit CM8 may also include one or more sensor devices 8d designed to determine the angular position αi of the pedal-crank 4 instant by instant. The sensor devices 8d may include, for example, a gyroscope and/or magnets.

The electronic power meter apparatus 8 may also include a memory device 8e and a processing device 8f designed to receive and store, in the memory device 8e, measurement data including the following measurement quantities: the force(s) measured by the sensor devices 8b, the angular speed ω of the pedal-crank 4 measured by the sensor devices 8c, and the angular position αi of the pedal-crank 4 measured by the sensor devices 8d. Preferably, the above measurement quantities can be measured by the relevant sensor devices and stored in the memory device 8e by the processing device 8f continuously at predetermined instants and/or predetermined angles when the pedal-crank 4 rotates around the axis A. Conveniently, the above measurement quantities included in the measurement data can be measured and processed to calculate respective "mean" values at predetermined angular intervals of rotation of the pedal crank 4, e.g., every about 5°.

The processing device 8f may also be configured to: receive, as input, the command signal S1 to determine the pedalling arc AP defined and/or set by the user, and process the measurement data determined in the pedalling arc AP set and/or selected by the user, so as to conveniently provide the pedalling performance data specifically (exclusively) associated with the pedalling arc AP itself.

According to a preferred embodiment, the processing device 8f is conveniently configured to implement a first metric to determine first pedalling performance data indicative of the useful power or propulsive power PP(AP) generated by the user in the pedalling arc AP set by the user. The propulsive power PP(AP) can be calculated by the processing device 8f at least on the basis of the effective force FR(AP) and angular speed ω(AP) measured by the measurement circuit CM8 in the pedalling arc AP (of the pedal 2) set by the user.

Preferably, the processing device 8f is conveniently configured to determine a first performance profile containing the propulsive powers PP(AP) generated by the user in the set pedalling arc AP (of the pedal 2), which were stored during a plurality of revolutions of the pedal-crank 4 around the axis A. The first performance profile can therefore be conveniently indicative of the trend of the propulsive power PP(AP) generated by the user in a pedalling arc AP set by the user during the pedal strokes performed by the user, for example, in a training cycle (multiple pedal strokes).

According to a preferred embodiment, the processing device 8f is conveniently configured to implement a second metric to determine second pedalling performance data indicative of the user's force effectiveness EF in the pedalling arc AP set by the user. The force effectiveness EF(AP) can be calculated by the processing device 8f using the formula EF=FR(AP)/FT(AP) on the basis of the effective force FR(AP) and resultant force FT(AP) measured by the measurement circuit in the pedalling arc AP set by the user.

Preferably, the processing device 8f is conveniently configured to determine a second performance profile containing the user's force effectiveness EF(AP) in the pedalling arc AP set by the user, which was determined during a plurality of pedal strokes. The second performance profile can therefore be indicative of the trend of the user's force effectiveness profile in the same pedalling arc AP of the pedal 2 during the pedal strokes performed by the user, for example, in a training cycle.

According to a preferred embodiment, the processing device 8f is conveniently configured to implement a third metric to determine third pedalling performance data indicative of the user' index of effectiveness IE(AP) in the pedalling arc AP set by the user. The index of effectiveness IE(AP) can be calculated by the processing device 8f using the formula IE(AP)=EF(AP)/FT(AP) based on the ratio of the force effectiveness EF(AP) in the pedalling arc AP (of the pedal 2) set by the user to the resultant force FT(AP) in the pedalling arc AP set by the user.

Preferably, the processing device 8f is conveniently configured to determine a third performance profile containing the user's index of effectiveness IE(AP) in the pedalling arc AP set by the user when the pedal-crank makes a plurality of full revolutions around the axis A. The third performance profile can therefore be conveniently indicative of the trend of the user's index of effectiveness IE(AP) during the pedal strokes performed by the user, for example, in a training cycle (multiple pedal strokes).

According to a preferred embodiment, the processing device 8f is conveniently configured to selectively store the first performance data and/or the second performance data and/or the third performance data, only when a predetermined pedalling condition occurs/is met during one pedalling revolution. This predetermined pedalling condition can conveniently occur/be met, for example, when: the propulsive power PP is within a predetermined power range, and/or the cadence of the pedal-crank 4 is within a predetermined cadence range, and/or the user is in a predetermined postural condition (for example sitting or standing).

According to a preferred embodiment, the user interface device 7 is designed to allow the user, by sending command data contained in the command signal S1, to command the power meter apparatus 8 to implement, determine, and provide: the first metric and/or the second metric and/or the third metric, and/or the first performance profile and/or the second performance profile and/or the third performance profile.

According to a preferred embodiment, the user interface device 7 is also designed to allow the user, by sending command data contained in the command signal S1, to select/command the predetermined pedalling condition for the selective storage of the first performance data, and/or the second performance data, and/or the third performance data.

The processing device 8f of the power meter apparatus 8 is conveniently configured to implement/determine, based on the command data contained in the command signal S1: the first metric and/or the second metric and/or the third metric, and/or the first performance profile, and/or the second performance profile, and/or the third performance profile.

The processing device 8f of the power meter apparatus 8 is conveniently configured to implement, based on the command data of the command signal S1, the selective storage of the first performance data, and/or the second performance data, and/or the third performance data, based on the pedalling condition established by the user.

In addition, the processing device 8f of the power meter apparatus 8 is configured to provide the user interface device 7, via the communication unit 8a, with performance data relating to: the first metric and/or the second metric and/or the third metric, and/or the first performance profile, and/or the second performance profile, and/or the third performance profile.

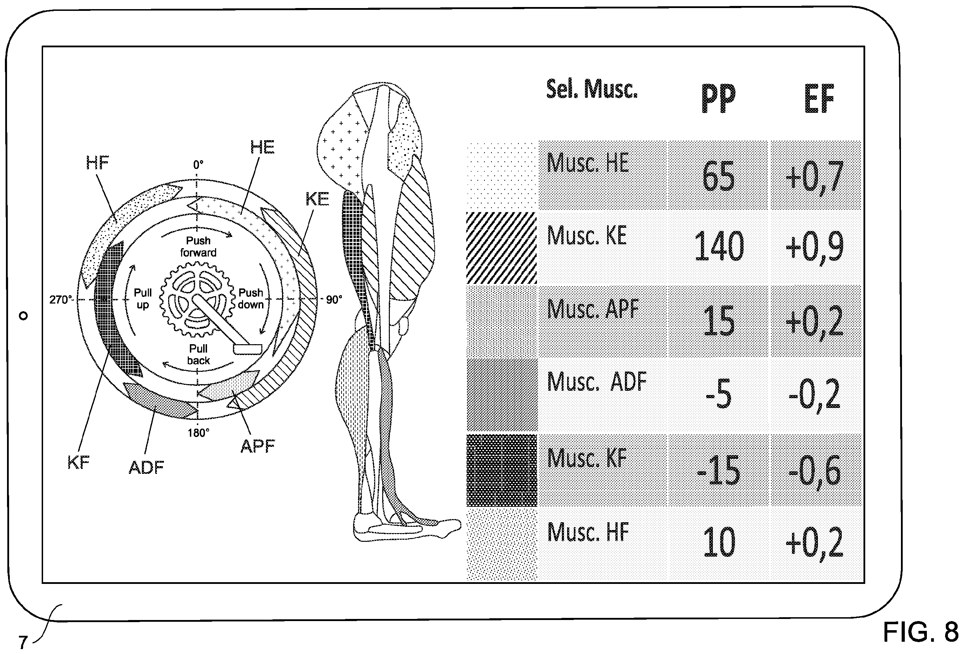

Referring to Figure 8, additionally or alternatively, the user interface device 7 can be configured to provide command data in the command signal S1 containing the pedalling arc AP, in turn determined on the basis of a prior selection by a user of a muscle group M1 of the lower limb that moves the pedal 2.

According to this embodiment, the user interface device 7 can be configured to: allow a user to select at least one muscle group of said lower limb included in a group of predetermined muscle organs associated with respective pedalling arcs AP, and provide command data in the command signal S1 containing the pedalling arc AP associated with the selected muscle group. In other words, the pedalling arc AP can be selected indirectly, that is, based on a user command indicated with M1 in Figure 2 indicating a muscle group. In this embodiment, the memory unit 7e contains a series of predetermined pedalling arcs AP and, for each pedalling arc AP, a respective muscle group.

In the example shown in Figure 8, the selection of a muscle group related to the hip extensors (indicated with HE in Figure 8) automatically causes the setting, i.e., the selection, of a pedalling arc AP between α1=0° and α2=70°; the selection of a muscle group associated with the knee extensors (indicated with KE in Figure 8) automatically causes the setting, i.e., the selection, of a pedalling arc AP between α1=70° and α2=150°; the selection of a muscle group associated with the ankle plantar flexors (indicated with APF in Figure 8) automatically causes the setting, i.e., the selection, of a pedalling arc AP between α1= 150° and α2= 180°; the selection of a muscle group associated with the ankle dorsiflexors (indicated with ADF in Figure 8) automatically causes the setting, i.e., the selection, of a pedalling arc AP between α1=180° and α2= 220°; the selection of a muscle group associated with the knee flexors (indicated with KF in Figure 8) automatically causes the setting, i.e., the selection, of a pedalling arc AP between α1=220° and α2=290°; the selection of a muscle group associated with the hip flexors (indicated with HF in Figure 8) automatically causes the setting, i.e., the selection, of a pedalling arc AP between α1=290° and α2=360°.

The technical effect obtained is to enable a user to verify the performance of specific muscle organs during pedalling by monitoring the force exerted exclusively in the pedalling arcs AP in which these same muscle groups are activated. In other words, the performance data of the pedalling arc AP are indicative of the performance of the muscle group associated with the pedalling arc AP itself.

It is understood that the present invention is not limited to the selection of a single pedalling arc AP and/or muscle group but may include, additionally or alternatively, the setting or selection by the user of a series of pedalling arcs AP and/or of muscle organs. In this case, the electronic system 1 is designed to selectively provide the performance data determined by the power meter apparatus for each pedalling arc determined directly or indirectly (through selection of the muscle group) by the user via the user interface device 7.

It is also understood that the user interface device 7 can be configured so that the user can selectively set (select and/or define) pedalling arcs AP that are at least partially overlapping. In this case, the power meter apparatus 8 is configured to provide performance data for each set (selected) pedalling arc AP.

It is also understood that the user interface device 7 can be configured to allow the user to set and/or select and/or define metrics that include performance data of a selected pedalling arc AP, which are different from another selected pedalling arc AP.

It is also understood that the electronic system 1, according to one embodiment, may provide that the power meter apparatus 8 selectively only stores in the memory device 8e the measurement quantities determined in the pedalling arc AP and communicates these measurement quantities to the user interface device. According to this embodiment, the processing carried out by the processing device 8f may be carried out by the processing unit 7d. The technical effect of this embodiment is to achieve a reduction in the computing power required by the processing device 8f.

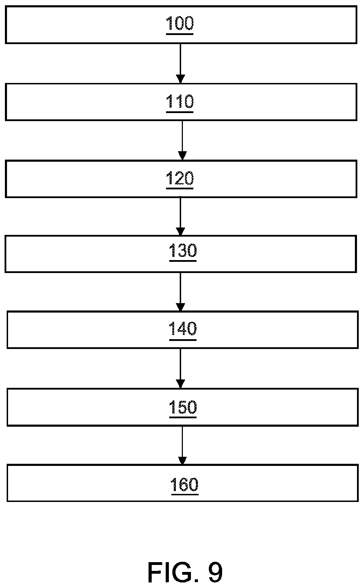

With reference to Figure 9, the following describes the operating steps of the method of operation of the electronic measuring system 1 described above, assuming the selection of a single pedalling arc AP.

The user sets, e.g., selects, via the interface device 7, a pedalling arc (block 100). The setting, i.e., the selection of the pedalling arc AP, can be done, e.g., selectively, directly on the basis of a series of predetermined pedalling arcs AP or by defining/adjusting the geometrical parameters characterizing the pedalling arc AP (Figure 5).

It is understood that the setting or selection of the pedalling arc AP can be done, for example, indirectly, i.e., on the basis of a user command indicative of the muscle group selected by the user (Figure 8). In this case, the user interface device 7 automatically determines (sets) the pedalling arc AP based on the muscle group selected by the user.

The user, via the interface device 7, can also set or establish/select the request to be displayed: the first metric, and/or the second metric, and/or the third metric, and/or the first performance profile, and/or the second performance profile and/or the third performance profile (block 110).

The user may also set or determine the pedalling condition that will be used by the power meter apparatus 8 to selectively store the first performance data and/or the second performance data and/or the third performance data (block 120).

The power meter apparatus 8 receives the user-command signal S1 and determines the pedalling arc AP.

The power meter apparatus 8 measures the force(s) in the pedalling arc AP and determines the measurement quantities (block 130).

The power meter apparatus 8 processes the measurement quantities determined in the pedalling arc AP and, based thereon and on the command data contained in the received command signal S1, determines one or more of the following performance data: the first metric and/or the second metric and/or the third metric, and/or the first performance profile, and/or the second performance profile, and/or the third performance profile (block 140).

The storage of the first performance data, and/or the second performance data, and/or the third performance data may be conveniently done selectively on the basis of the pedalling condition established by the user.

The power meter apparatus 8 communicates (transmits) the user's performance data determined in the pedalling arc AP to the user interface device 7 (block 150). The power meter apparatus 8, through the communication system 100, can communicate to the user interface device 7, on the basis of the setting or selection made by the user, performance data relating to: the first metric and/or the second metric and/or the third metric, and/or the first performance profile, and/or the second performance profile, and/or the third performance profile.

The user interface device 7 communicates to the user, preferably by display, the performance data received from the power meter apparatus 8 (block 160).

The system described above advantageously allows a cyclist to train in a way that improves the performance data in a specific pedalling phase and sector, for example, when the leg is lifted to counteract its weight during the rise of the pedal. The user can then advantageously set a pedalling arc, for example between 220° and 320°, view in real time the performance data in that set pedalling arc and correct his/her movement, again in real time, to increase his/her performance. If, on the other hand, the user needs to improve the performance data when the pedal is pushed backwards, i.e., in the lower part of the pedalling cycle, he/she can select a pedalling arc between 140° and 220°. In this case, too, the user obtains the performance data in real time and can adjust his/her movement in real time to increase the performance when pushing within the set pedalling arc.

In other words, a technical effect achieved by enabling the user to select and set a single pedalling arc is to enable the user to determine precisely, according to his/her own needs, the specific sector of interest contained in the pedalling circumference.

This technical problem is solved by the present solution since, as it is possible to establish a single sector of interest and view the data from that sector alone, it makes it easier for the cyclist to control the situation even in critical high-effort conditions in which the information provided to the cyclist must be immediately available for consultation. Tests carried out by the Applicant have shown that a professional cyclist in some critical conditions has difficulty in examining a lot of data, such as, for example, a power distribution over the entire pedalling circumference. Therefore, a technical advantage is to provide the cyclist with the useful data to be observed for the set sector in a simplified and immediate way.

Moreover, a further technical advantage of the present invention is to allow the cyclist to select the sector at will according to the level of refinement to be achieved (the sector could even be about ten degrees) so that data can be easily accessed for that "specific sector", thus eliminating from his/her consultation the "noise" of the data that is of no interest to him/her.

In addition, if the user wishes to monitor the performance data for an entire training cycle, for example, the "useful power" or the "force effectiveness" over a given pedalling arc, he/she can view/download the stored performance profiles so that a technical analysis can be made on the basis of the parameters of interest within the set pedalling arc.

Furthermore, the system provides useful information for evaluating the performance of specific muscle groups of the athlete that are activated in specific pedalling arcs in order to highlight any shortcomings, so that specific exercises can be planned to selectively improve the muscle groups.

The pedalling arc-related performance data determined by the system may be examined by a biomechanic or athletic trainer in order to identify the muscle groups that need to be specifically trained. Storing performance profiles over time allows comparisons to be made with previous profiles in order to detect improvement/worsening in the performance.

## Claims

1. An electronic measuring system (1) configured to determine performance-data of a user relating to one or more pedal-strokes exercised by the user on a pedal (2) of a bicycle (3),
said bicycle (3) is provided with two pedal-cranks (4) designed to rotate around a rotation axis (A), and two pedals (2) which are coupled to said respective two pedal-cranks (4) so that each one describes a pedalling circumference (CP) during the complete rotation of the corresponding pedal-crank (4) around said rotation axis (A),
the electronic measuring system (1) comprises:
a user interface device (7) which is configured to allow a user to set a pedalling-arc (AP) comprised in said pedalling circumference (CP) of said pedal (2), provide a user-command (S1) containing said pedalling-arc (AP) set by the user, and
a power meter apparatus (8) which is configured to:
be operationally connected to said user interface device (7) to receive said user-command (S1) containing the pedalling-arc (AP) set by said user,
measure at least one force exerted by the said user during pedalling, when the pedal (2) is moving in the said pedalling-arc (AP) set by said user,
determine at least one measurement quantity associated with said force measured in said pedalling-arc (AP) set by said user, and
determine the performance data of said user in said pedalling-arc (AP) based on said determined measurement quantity, **characterized in that** said pedalling-arc (AP) does not have a predetermined fixed value.

2. The electronic measuring system according to claim 1, wherein said power meter apparatus (8) is further configured to provide the user interface device (7) with said performance data of said user determined in said pedalling-arc (AP) set by the user; said user interface device (7) is configured to communicate to said user said performance data determined in said pedalling-arc (AP) set by the user.

3. The electronic measuring system according to claims 1 or 2, wherein said user interface device (7) is configured to: allow a user to select at least one muscle group, automatically set a pedalling-arc (AP) based on said muscle group selected by the user, and provide said power meter apparatus (8) with said user-command (S1) containing said determined pedalling-arc (AP).

4. The electronic measuring system according to claim 3, comprising a memory unit (7e) containing a series of pedalling arcs (AP) of the pedal (2) and, for each pedalling arc (AP) of the pedal (2), a respective muscle group of the leg operating the pedal (2).

5. The electronic measuring system according to any one of the foregoing claims, wherein said power meter apparatus (8) is further configured to perform at least one metric for determining performance data comprising: the propulsive power (PP) and/or the force effectiveness (EF) and/or the index of effectiveness (IE) of the said user in the said pedalling arc (AP) set by the user.

6. The electronic measuring system according to claim 5, wherein said power meter apparatus (8) is configured to determine a performance profile of said user in said pedalling-arc (AP) set by the user; said performance profile comprises said performance data determined in the pedalling-arc (AP) set by the user when the pedal-crank makes a plurality of full revolutions around said rotation axis (A).

7. The electronic measuring system according to any of the foregoing claims, wherein said power meter apparatus (8) is further configured to determine said performance data only within the said pedalling arc (AP) set by the user, without the measurement quantities determined on the basis of the force which is measured when the said pedal (2) is moving in the said pedalling circumference (CP) outside said pedalling-arc (AP) set by the user.

8. The electronic measuring system according to any of the foregoing claims, wherein said power meter apparatus (8) is mounted on a pedal (2) to measure the force exerted by the user on said pedal (2).

9. The electronic measuring system according to claim 1, wherein said user interface device (7) is configured to allow a user to adjust/vary said pedalling arc (AP).

10. The electronic measuring system according to any of the foregoing claims, comprising a processing device configured to implement a first metric to determine first pedalling performance data indicative of the useful power or propulsive power (PP(AP)) generated by the user in said pedalling-arc (AP) set by the user.

11. The electronic measuring system according to any of the foregoing claims, comprising a processing device configured to implement a second metric to determine second pedalling performance data indicative of the user's force effectiveness (EF) in said pedalling-arc (AP) of the pedal (2) set by said user.

12. The electronic measuring system according to any of the foregoing claims, comprising a processing device configured to implement a third metric to determine third pedalling performance data indicative of the user's index of effectiveness (IE(AP)) in said pedalling-arc (AP) set by the user.

13. A method for determining performance data of a user relating to the pedal-stroke exercised by the user on a pedal (2) of a bicycle (3),
said bicycle (3) is provided with two pedal-cranks (4) designed to rotate around a rotation axis (A), and two pedals (2) which are coupled to said respective two pedal-cranks (4) so that each one describes a pedalling circumference (CP) during the complete rotation of the corresponding pedal-crank (4) around said rotation axis (A),
the method comprises the steps of:
setting, by means of a user interface device (7), a pedalling-arc (AP) comprised in said pedalling circumference (CP) wherein said pedalling-arc (AP) does not have a predetermined fixed value and providing a user-command (S1) containing said set pedalling-arc (AP),
receiving, through said power meter apparatus (8), said user-command (S1) containing the pedalling-arc (AP) set by said user,
measuring, by means of a power meter apparatus (8), at least one force exerted by the said user during pedalling, when the pedal (2) is moving in a pedalling-arc (AP) set by said user,
determining, by means of said power meter apparatus (8), at least one measurement quantity associated with said force measured when the pedal (2) is moving in said set pedalling-arc (AP), and
determining, by means of said power meter apparatus (8), the performance data of said user in said pedalling-arc (AP) set by the user, based on said determined quantity.

14. The method according to claim 13, comprising the steps of providing through said power meter apparatus (8) said performance data relating to said pedalling-arc (AP), set by the user, to the user interface device (7), and communicating said performance data to the user via said user interface device (7).

15. The method according to claims 13 or 14, comprising the steps of selecting a muscle group through said user interface device (7),
automatically setting a pedalling-arc (AP) on the basis of the selected muscle group, and providing said power meter apparatus (8) with the user-command (S1) containing said automatically set pedalling-arc (AP).

## Patentansprüche

1. Elektronisches Messsystem (1), das konfiguriert ist, Leistungsdaten eines Benutzers in Bezug auf einen oder mehrere Pedalhübe bzw. -tritte zu bestimmen, die von dem Benutzer auf einem Pedal (2) eines Fahrrads (3) ausgeführt werden,
wobei das Fahrrad (3) mit zwei Tret- bzw. Pedalkurbeln (4), die ausgelegt sind, sich um eine Drehachse (A) zu drehen, und mit zwei Pedalen (2) versehen ist, die mit den jeweiligen zwei Pedalkurbeln (4) gekoppelt sind, so dass jede während der vollständigen Drehung der entsprechenden Pedalkurbel (4) um die Drehachse (A) einen Pedal- bzw. Tretumfang (CP) beschreibt,
wobei das elektronische Messsystem (1) umfasst:
eine Benutzerschnittstellenvorrichtung (7), die konfiguriert ist, einem Benutzer zu ermöglichen, einen Pedal- bzw. Tretbogen (AP) einzustellen, der in dem Tretumfang (CP) des Pedals (2) umfasst ist, und einen Benutzerbefehl (S1) bereitzustellen, der den von dem Benutzer eingestellten Tretbogen (AP) enthält, und
eine Leistungsmessvorrichtung (8), die konfiguriert ist:
operativ mit der Benutzerschnittstellenvorrichtung (7) verbunden zu sein, um den Benutzerbefehl (S1) zu empfangen, der den von dem Benutzer eingestellten Tretbogen (AP) enthält,
zumindest eine Kraft zu messen, die von dem Benutzer während des Pedaltretens ausgeübt wird, wenn sich das Pedal (2) in dem von dem Benutzer eingestellten Tretbogen (AP) bewegt,
zumindest eine Messgröße zu bestimmen, die mit der Kraft verknüpft ist, die in dem von dem Benutzer eingestellten Tretbogen (AP) gemessen wird, und
die Leistungsdaten des Benutzers in dem Tretbogen (AP) basierend auf der bestimmten Messgröße zu bestimmen,
**dadurch gekennzeichnet, dass** der Tretbogen (AP) keinen vorbestimmten festen Wert aufweist.

2. Elektronisches Messsystem nach Anspruch 1, wobei die Leistungsmessvorrichtung (8) ferner konfiguriert ist, der Benutzerschnittstellenvorrichtung (7) die in dem von dem Benutzer eingestellten Tretbogen (AP) bestimmten Leistungsdaten des Benutzers bereitzustellen; wobei die Benutzerschnittstellenvorrichtung (7) konfiguriert ist, dem Benutzer Leistungsdaten zu übermitteln, die in dem von dem Benutzer eingestellten Tretbogen (AP) bestimmt werden.

3. Elektronisches Messsystem nach Anspruch 1 oder 2, wobei die Benutzerschnittstellenvorrichtung (7) konfiguriert ist: einem Benutzer zu ermöglichen, zumindest eine Muskelgruppe auszuwählen, automatisch einen Tretbogen (AP) basierend auf der von dem Benutzer ausgewählten Muskelgruppe einzustellen und der Leistungsmessvorrichtung (8) den Benutzerbefehl (S1) bereitzustellen, der den bestimmten Tretbogen (AP) enthält.

4. Elektronisches Messsystem nach Anspruch 3, umfassend eine Speichereinheit (7e), die eine Reihe von Pedalbögen (AP) des Pedals (2) und für jeden Tretbogen (AP) des Pedals (2) eine jeweilige Muskelgruppe des Beins enthält, welches das Pedal (2) betätigt.

5. Elektronisches Messsystem nach einem der vorhergehenden Ansprüche, wobei die Leistungsmessvorrichtung (8) ferner konfiguriert ist, zumindest eine Metrik zum Bestimmen von Leistungsdaten auszuführen, die umfassen: die Antriebsleistung (PP) und/oder die Kraftwirksamkeit (EF) und/oder den Wirksamkeitsindex (IE) des Benutzers in dem von dem Benutzer eingestellten Tretbogen (AP).

6. Elektronisches Messsystem nach Anspruch 5, wobei die Leistungsmessvorrichtung (8) konfiguriert ist, ein Leistungsprofil des Benutzers in dem von dem Benutzer eingestellten Tretbogen (AP) zu bestimmen; wobei das Leistungsprofil die Leistungsdaten umfasst, die in dem von dem Benutzer eingestellten Tretbogen (AP) bestimmt werden, wenn die Pedalkurbel eine Mehrzahl von vollen Umdrehungen um die Drehachse (A) macht.

7. Elektronisches Messsystem nach einem der vorhergehenden Ansprüche, wobei die Leistungsmessvorrichtung (8) ferner konfiguriert ist, die Leistungsdaten nur innerhalb des von dem Benutzer eingestellten Tretbogens (AP) zu bestimmen, ohne dass die Messgrößen auf der Basis der Kraft bestimmt werden, die gemessen wird, wenn sich das Pedal (2) in dem Tretumfang (CP) außerhalb des von dem Benutzer eingestellten Tretbogens (AP) bewegt.

8. Elektronisches Messsystem nach einem der vorhergehenden Ansprüche, wobei die Leistungsmessvorrichtung (8) an einem Pedal (2) montiert ist, um die von dem Benutzer auf das Pedal (2) ausgeübte Kraft zu messen.

9. Elektronisches Messsystem nach Anspruch 1, wobei die Benutzerschnittstellenvorrichtung (7) konfiguriert ist, einem Benutzer zu ermöglichen, den Tretbogen (AP) anzupassen/zu variieren.

10. Elektronisches Messsystem nach einem der vorhergehenden Ansprüche, umfassend eine Verarbeitungsvorrichtung, die konfiguriert ist, eine erste Metrik zu implementieren, um erste Pedalleistungsdaten zu bestimmen, welche die Nutzleistung oder Antriebsleistung (PP (AP)) angeben, die von dem Benutzer in dem von dem Benutzer eingestellten Tretbogen (AP) erzeugt wird.

11. Elektronisches Messsystem nach einem der vorhergehenden Ansprüche, umfassend eine Verarbeitungsvorrichtung, die konfiguriert ist, eine zweite Metrik zu implementieren, um zweite Pedalleistungsdaten zu bestimmen, welche die Kraftwirksamkeit (EF) des Benutzers in dem von dem Benutzer eingestellten Tretbogen (AP) des Pedals (2) angeben.

12. Elektronisches Messsystem nach einem der vorhergehenden Ansprüche, umfassend eine Verarbeitungsvorrichtung, die konfiguriert ist, eine dritte Metrik zu implementieren, um dritte Pedalleistungsdaten zu bestimmen, die den Wirksamkeitsindex (IE (AP)) des Benutzers in dem von dem Benutzer eingestellten Tretbogen (AP) angeben.

13. Verfahren zum Bestimmen von Leistungsdaten eines Benutzers in Bezug auf den Pedalhub bzw. -tritt, der von dem Benutzer auf einem Pedal (2) eines Fahrrads (3) ausgeführt wird,
wobei das Fahrrad (3) mit zwei Tret- bzw. Pedalkurbeln (4), die ausgelegt sind, sich um eine Drehachse (A) zu drehen, und mit zwei Pedalen (2) versehen ist, die mit den jeweiligen zwei Pedalkurbeln (4) gekoppelt sind, so dass jede während der vollständigen Drehung der entsprechenden Pedalkurbel (4) um die Drehachse (A) einen Tret- bzw. Pedalumfang (CP) beschreibt,
wobei das Verfahren die Schritte umfasst:
Einstellen, mittels einer Benutzerschnittstellenvorrichtung (7), eines in dem Tretumfang (CP) umfassten Pedal- bzw. Tretbogens (AP), wobei der Tretbogen (AP) keinen vorbestimmten festen Wert aufweist, und Bereitstellen eines Benutzerbefehls (S1), der den eingestellten Tretbogen (AP) enthält,
Empfangen, durch die Leistungsmessvorrichtung (8), des Benutzerbefehls (S1), der den von dem Benutzer eingestellten Tretbogen (AP) enthält,
Messen, mittels einer Leistungsmessvorrichtung (8), zumindest einer Kraft, die von dem Benutzer während des Tretens ausgeübt wird, wenn sich das Pedal (2) in einem von dem Benutzer eingestellten Tret- bzw. Tretbogen (AP) bewegt, Bestimmen, mittels der Leistungsmessvorrichtung (8), zumindest einer Messgröße, die mit der Kraft verknüpft ist, die gemessen wird, wenn sich das Pedal (2) in dem eingestellten Tretbogen (AP) bewegt, und
Bestimmen, mittels der Leistungsmessvorrichtung (8), der Leistungsdaten des Benutzers in dem von dem Benutzer eingestellten Tretbogen (AP) basierend auf der bestimmten Größe.

14. Verfahren nach Anspruch 13, umfassend die Schritte Bereitstellen, durch die Leistungsmessvorrichtung (8), der Leistungsdaten in Bezug auf den von dem Benutzer eingestellten Tretbogen (AP) an die Benutzerschnittstellenvorrichtung (7) und Übermitteln der Leistungsdaten an den Benutzer über die Benutzerschnittstellenvorrichtung (7).

15. Verfahren nach Anspruch 13 oder 14, umfassend die Schritte Auswählen einer Muskelgruppe durch bzw. über die Benutzerschnittstellenvorrichtung (7),
automatisches Einstellen eines Pedal- bzw. Tretbogens (AP) auf der Basis der ausgewählten Muskelgruppe und Bereitstellen des Benutzerbefehls (S1), der den automatisch eingestellten Tretbogen (AP) enthält, an die Leistungsmessvorrichtung (8).

## Revendications

1. Système de mesure électronique (1) configuré pour déterminer des données de performance d'un utilisateur relatives à un ou plusieurs coups de pédale exercés par l'utilisateur sur une pédale (2) d'un vélo (3),
ledit vélo (3) étant pourvu de deux manivelles de pédale (4) conçues pour tourner autour d'un axe de rotation (A), et de deux pédales (2) qui sont couplées auxdites deux manivelles de pédale (4) respectives de sorte que chacune d'elles décrive une circonférence de pédalage (CP) pendant la rotation complète de la manivelle de pédale (4) correspondante autour dudit axe de rotation (A),
le système de mesure électronique (1) comprend :
un dispositif d'interface utilisateur (7) qui est configuré pour permettre à un utilisateur de définir un arc de pédalage (AP) compris dans ladite circonférence de pédalage (CP) de ladite pédale (2), et
pour fournir une commande utilisateur (S1) contenant ledit arc de pédalage (AP) défini par l'utilisateur, et
un appareil de mesure de puissance (8) qui est configuré pour :
être connecté de manière opérationnelle audit dispositif d'interface utilisateur (7) pour recevoir ladite commande utilisateur (S1) contenant l'arc de pédalage (AP) défini par ledit utilisateur,
mesurer au moins une force exercée par ledit utilisateur pendant le pédalage, lorsque la pédale (2) se déplace dans ledit arc de pédalage (AP) défini par ledit utilisateur,
déterminer au moins une quantité de mesure associée à ladite force mesurée dans ledit arc de pédalage (AP) défini par ledit utilisateur, et
déterminer les données de performance dudit utilisateur dans ledit arc de pédalage (AP) sur la base de ladite quantité de mesure déterminée, **caractérisé en ce que** ledit arc de pédalage (AP) n'a pas de valeur fixe prédéterminée.

2. Système de mesure électronique selon la revendication 1, dans lequel ledit appareil de mesure de puissance (8) est en outre configuré pour fournir au dispositif d'interface utilisateur (7) lesdites données de performance dudit utilisateur déterminées dans ledit arc de pédalage (AP) défini par l'utilisateur ; ledit dispositif d'interface utilisateur (7) est configuré pour communiquer audit utilisateur lesdites données de performance déterminées dans ledit arc de pédalage (AP) défini par l'utilisateur.

3. Système de mesure électronique selon la revendication 1 ou 2, dans lequel ledit dispositif d'interface utilisateur (7) est configuré pour : permettre à un utilisateur de sélectionner au moins un groupe musculaire, définir automatiquement un arc de pédalage (AP) sur la base dudit groupe musculaire sélectionné par l'utilisateur, et fournir audit appareil de mesure de puissance (8) ladite commande utilisateur (S1) contenant ledit arc de pédalage (AP) déterminé.

4. Système de mesure électronique selon la revendication 3, comprenant une unité de mémoire (7e) contenant une série d'arcs de pédalage (AP) de la pédale (2) et, pour chaque arc de pédalage (AP) de la pédale (2), un groupe musculaire respectif de la jambe actionnant la pédale (2).

5. Système de mesure électronique selon l'une quelconque des revendications précédentes, dans lequel ledit appareil de mesure de puissance (8) est en outre configuré pour effectuer au moins une mesure pour déterminer des données de performance comprenant : la puissance propulsive (PP) et/ou l'efficacité de force (EF) et/ou l'indice d'efficacité (IE) dudit utilisateur dans ledit arc de pédalage (AP) défini par l'utilisateur.

6. Système de mesure électronique selon la revendication 5, dans lequel ledit appareil de mesure de puissance (8) est en outre configuré pour déterminer un profil de performance dudit utilisateur dans ledit arc de pédalage (AP) défini par l'utilisateur, ledit profil de performance comprenant lesdites données de performance déterminant dans l'arc de pédalage (AP) défini par l'utilisateur lorsque la manivelle de pédale effectue une pluralité de tours complets autour dudit axe de rotation (A).

7. Système de mesure électronique selon l'une quelconque des revendications précédentes, dans lequel ledit appareil de mesure de puissance (8) est en outre configuré pour déterminer lesdites données de performance uniquement dans ledit arc de pédalage (AP) défini par l'utilisateur sans que les quantités de mesure déterminées sur la base de la force soient mesurées lorsque ladite pédale (2) se déplace dans ladite circonférence de pédalage (CP) en dehors dudit arc de pédalage (AP) défini par l'utilisateur.

8. Système de mesure électronique selon l'une quelconque des revendications précédentes, dans lequel ledit appareil de mesure de puissance (8) est monté sur une pédale (2) pour mesurer la force exercée par l'utilisateur sur ladite pédale (2).

9. Système de mesure électronique selon la revendication 1, dans lequel ledit dispositif d'interface utilisateur (7) est configuré pour permettre à un utilisateur de régler/varier ledit arc de pédalage (AP).

10. Système de mesure électronique selon l'une quelconque des revendications précédentes, comprenant un dispositif de traitement configuré pour mettre en œuvre une première métrique pour déterminer des premières données de performance de pédalage indicatives de la puissance utile ou de la puissance propulsive (PP (AP)) générée par l'utilisateur dans ledit arc de pédalage (AP) défini par l'utilisateur.

11. Système de mesure électronique selon l'une quelconque des revendications précédentes, comprenant un dispositif de traitement configuré pour mettre en œuvre une deuxième métrique pour déterminer des deuxièmes données de performance de pédalage indicatives de l'efficacité de force (EF) de l'utilisateur dans ledit arc de pédalage (AP) de la pédale (2) définie par ledit utilisateur.

12. Système de mesure électronique selon l'une quelconque des revendications précédentes, comprenant un dispositif de traitement configuré pour mettre en œuvre une troisième métrique pour déterminer des troisièmes données de performance de pédalage indicatives de l'indice d'efficacité (IE (AP)) de l'utilisateur dans ledit arc de pédalage (AP) défini par l'utilisateur.

13. Procédé de détermination de données de performance d'un utilisateur relatives au coup de pédale exercé par l'utilisateur sur une pédale (2) d'un vélo (3), ledit vélo (3) étant pourvu de deux manivelles de pédale (4) conçues pour tourner autour d'un axe de rotation (A), et de deux pédales (2) qui sont couplées auxdites deux manivelles de pédale (4) respectives de sorte que chacune d'elles décrive une circonférence de pédalage (CP) pendant la rotation complète de la manivelle de pédale (4) correspondante autour dudit axe de rotation (A),
le procédé comprend les étapes consistant à :
définir, au moyen d'un dispositif d'interface utilisateur (7), un arc de pédalage (AP) compris dans ladite circonférence de pédalage (CP) dans lequel ledit arc de pédalage (AP) n'a pas de valeur fixe prédéterminée et fournir une commande utilisateur (S1) contenant ledit arc de pédalage (AP) défini,
recevoir, par l'intermédiaire dudit appareil de mesure de puissance (8), ladite commande utilisateur (S1) contenant l'arc de pédalage (AP) défini par ledit utilisateur,
mesurer, au moyen d'un appareil de mesure de puissance (8) au moins une force exercée par ledit utilisateur pendant le pédalage, lorsque la pédale (2) se déplace dans un arc de pédalage (AP) défini par ledit utilisateur,
déterminer, au moyen dudit appareil de mesure de puissance (8), au moins une grandeur de mesure associée à ladite force mesurée lorsque la pédale (2) se déplace dans ledit arc de pédalage (AP) défini, et
déterminer, au moyen dudit appareil de mesure de puissance (8), les données de performance dudit utilisateur dans ledit arc de pédalage (AP) défini par l'utilisateur, sur la base de ladite grandeur déterminée.

14. Procédé selon la revendication 13, comprenant les étapes consistant à fournir par l'intermédiaire dudit appareil de mesure de puissance (8) lesdites données de performance relatives audit arc de pédalage (AP), défini par l'utilisateur, au dispositif d'interface utilisateur (7) et à communiquer lesdites données de performance à l'utilisateur via ledit dispositif d'interface utilisateur (7).

15. Procédé selon les revendications 13 ou 14, comprenant les étapes consistant à sélectionner un groupe musculaire par l'intermédiaire dudit dispositif d'interface utilisateur (7), à définir automatiquement un arc de pédalage (AP) sur la base du groupe musculaire sélectionné, et à fournir audit appareil de mesure de puissance (8) la commande utilisateur (S1) contenant ledit arc de pédalage (AP) défini automatiquement.
